# EUROPEAN PATENT SPECIFICATION

(11) **EP 2 017 351 B1**
(45) Date of publication and mention of the grant of the patent: **27.10.2010**
(21) Application number: 07112893.8
(22) Date of filing: 20.07.2007
(51) Int. Cl.: C12Q 1/00, C12Q 1/28, G01N 33/543, G01N 33/573

(54) **Combined method for the sequential measurement of (1) the enzymatically active fraction and (2) the total amount of an enzyme**
Kombination aus Verfahren zur sequentiellen Messung (1.) der enzymatisch aktiven Fraktion und (2.) der Gesamtmenge eines Enzyms
Procédé combiné pour la mesure séquentielle de (1) la fraction active enzymaticalle et (2) la quantité totale d'une enzyme

(43) Date of publication of application: 21.01.2009
(73) Proprietor: Université de Liège, 4000 Liège (BE)
(72) Inventor: Serteyn, Didier, 4163 Tavier (BE); Dupont, Ginette, 4020 Liège (BE); Franck, Thierry, 4350 Remicourt (BE); Kohnen, Stephan, 4031 Angleur (BE)
(74) Representative: pronovem

(56) References cited:
- WO-A-2005/075986
- US-A1- 2005 239 143
- ICHIBANGASE TOMOKO ET AL: "Study on immunocapture-chemiluminescence assay of lipase activity in a biological sample." LUMINESCENCE : THE JOURNAL OF BIOLOGICAL AND CHEMICAL LUMINESCENCE 2006 JAN-FEB, vol. 21, no. 1, January 2006 (2006-01), pages 62-66, XP002455123 ISSN: 1522-7235

## Description

### Field of the invention

The present invention is related to a combined method for the sequential measurement of the enzymatically active fraction and the total amount of an enzyme [(such as myeloperoxidase (MPO)] in a sample, and that find improved applications in veterinary and human health fields.

### Background of the invention

The international patent application W02005/075986 describes a method and kit for the measurement of neutrophil cells activation.

This method is based upon either an ELISA or a SIEFED detection method and kit for measuring the activation status of neutrophil cells in a biological sample obtained from a mammal, preferably a horse.

This method could be used for the detection and/or the prediction of diseases or pathologies, to follow up the neutrophil cells activation during therapy of a diseased mammal, to evaluate the ability of neutrophil cells during their fight against micro-organisms and/or to destroy them, to evaluate the efficiency of immunomodulators (or the in vitro inhibitory capacity of drugs) by comparing the neutrophils cells activation status of treated and non treated neutrophils, to evaluate the ability of neutrophil cells treated with said modulator and/or drug to fight against microorganisms and/or to destroy them, to evaluate the natural defense capacity or ability of a mammal to fight against micro organisms and to screen or to select compounds which interact with myeloperoxidase (MPO).

The international Patent Application W02005/075986 describes also the use of both ELISA and SIEFED methods and kits to distinguish between total and active myeloperoxidase (MPO content). However, this document does not describe and does not suggest that MPO detection could be obtained by these methods in a combined bioassay or method upon the same solid support with the same binding antibody or portion of antibody.

### Aims of the invention

The present invention aims to provide a method that does not present the drawbacks of the state of the art and that allows the measurement of the active fraction and the total amount of an enzyme on the same sample, in a sequential manner with the same primary antibody upon the same solid support.

A main aim of the present invention is to provide a method to be used for a combined measurement of the enzymatically active fraction and the total amount of various types of enzymes that are involved in various pathologies, such as cardiac diseases and cancer, preferably enzymes which are involved in arteriosclerosis.

A further aim of the present invention is to provide such detection method which reduces detection delays and the number of used reactive compounds (especially the number of primary antibodies used, the number of solid support used, the number of purified enzyme for obtaining the calibration curve).

A last aim of the present invention is to propose such method which allows an automatisation of this method, particularly allowing a simultaneous activation measurement of the active fraction and the total amount of various types of enzymes, possibly presented simultaneously in the same biological sample.

### Definitions

The acronym ELISA stands for "Enzyme-Linked Immuno Sorbent Assay" and the acronym SIEFED stands for "Specific Immunological Extraction Followed by Enzymatic Detection".

"A sequential measurement of the enzymatically active fraction and the total amount of an enzyme present in a biological sample" means a detection method which, after the binding of the enzyme on the immobilized primary antibody allows a measurement of the enzymatically active fraction of an enzyme (i.e. the detection is performed upon active enzyme by the measurement of its enzymatic activity with the addition of adequate substrate and cosubstrate) followed by the measurement of the total amount of the enzyme which is applied upon the enzyme active or inactive. "A measurement" means a detection and possibly a quantification. The term 'an enzyme inhibitor' is a compound or agent that combines with an enzyme in such a manner as to prevent the normal substrate-enzyme combination and the catalytic reaction.

### Summary of the invention

The present invention is related to a combined method for the sequential measurement of the enzymatically active fraction and the total amount (active and possibly inactive amount) of an enzyme present in a biological sample, which is based upon a combined SIEFED-ELISA detection bioassay and method; such detection being performed after the capture of the enzyme (active and inactive) with the same primary antibody fixed upon the same solid support (surface), this primary antibody being directed against this enzyme. The inventors have discovered unexpectedly that these two methods (SIEFED-ELISA Method) (that seems to be antagonist to each other) can be combined efficiently and allow an efficient calibration upon the same calibration or standard curve with the use of the same primary antibody fixed upon a solid support. Furthermore, the inventors have observed unexpectedly that the same primary antibody fixed upon the same solid support could be used in these two combined steps. Indeed, the inventors have observed unexpectedly that the primary antibody fixed upon a solid support is not affected by any washing step used during the two methods and could be maintained upon the solid support with its bound enzyme.

As the sequential technique for myeloperoxidase (the SIEFED followed by the ELISA) is performed with the same primary antibodies coated on a multiwell plate, the method of the invention
- allows to economize primary antibodies,
- allows to economize solid support (multiwell plate),
- allows to economize the reference pure protein (myeloperoxidase) used to establish the standard (reference) curves,
- allows to save time : only one dilution of the samples, and only one at repartition of the samples in the wells are necessary.
Furthermore, the method of the invention
- allows to economize reagents (dilution buffer).
- assure a higher level of precision in the measurement of myeloperoxidase by SIEFED and by ELISA for a same sample (the primary antibodies and the reference or unknown sample are identical for the two measurements, variations due to little difference in the concentration of the antibodies or in the volume of the sample to be measured are excluded).
- this feature is important to establish a ratio total MPO/active MPO for a given sample.

The method according to the invention is an in vitro method for sequentially (by successive steps) measuring the enzymatically active fraction and the total amount of one or more enzyme(s) preferably myeloperoxidase (MPO), this method comprising the following successive steps of:
1) providing a biological sample, preferably a biological sample obtained from a mammal, such as a horse or a human, said sample containing the said enzyme(s) [or cells which may release the said enzyme(s)],
2) immunocapturing the said enzyme(s), present in the biological sample, by an enzyme specific (polyclonal or monoclonal) primary antibody or hyper-variable portion thereof, both being specific of this enzyme(s)and being fixed upon a solid support surface.
3) possibly removing by a (first) washing step any compound that interferes with the measurement of the activity of the enzyme immunocaptured by this primary antibody (or its portion).
4) obtaining from a SIEFED detection and quantification by detecting and/or measuring the enzyme activity, preferably by adding a specific substrate to be transformed by this enzyme into a visible (preferably a fluorescent) reaction product a measured enzyme activity value.
5) possibly comparing the said measured enzyme activity value with a normal enzyme activity value (i.e. standard of reference or preestablished standard enzyme values (levels) used as reference) obtained from a significant number (more than 10, preferably more than 50, more preferably more than 200 or 1000 individuals) of "healthy" mammals preferably horses or human (i.e. mammals that present normal enzyme values (levels) and do not suffer from the following described diseases or symptoms) or optionally quantifying enzyme activity levels (values) by using a standard enzyme curve,
6) possibly removing by a (second) washing step any substrate present upon the immunocapturated enzyme,
7) obtaining from an ELISA detection (ELISA step) a measured total enzyme amount (level or value), by detecting and measuring total amount (active and inactive) of the enzyme present in the biological sample, preferably by a (polyclonal or monoclonal) secondary antibody or hyper-variable portion thereof for the detection of the immunocapturated enzyme, (by the primary antibody or hyper-variable portion thereof), this said secondary antibody or portion thereof, both being specific of the immunocapturated enzyme and being possibly labeled by a coupling to a "detection" second enzyme being different from the first enzyme to be detected (labeled secondary antibody).
8) possibly comparing the said obtained measured total enzyme amount (levels or values) with normal enzyme amounts (levels or values) obtained from a significant number of "healthy" mammals, preferably healthy horses or human.
9) optionally quantifying the enzyme amount (levels or values) by using standard enzyme(s) curve(s)and
10) possibly relating the enzymatically active fractions of enzyme(s) measured by SIEFED, the total amount (levels or values) of the enzyme(s) measured by ELISA and the calculated rates of enzymatically active fraction to total amount (level or value) to an activation status of the enzyme (s) or the cells releasing the enzyme(s) and which is indicative of the presence, absence or condition of a disease or immunological status.

Also disclosed is a combined bioassay that comprises means and media (possibly present in a kit or device) for measuring an activation status of an enzyme(s) present in a biological sample or cells which may release the said enzyme in the biological sample (obtained from a mammal, preferably from a horse or a human) that allows the sequential measurement above described and a quantification upon a calibration or standard curve.

The combined bioassay (or kit or device) being a combined SIEFED-ELISA bioassay, (kit or device), to measure sequentially the enzymatically active fraction and the total (active and inactive) enzyme amount content, wherein the said fraction and content being correlated with a possible cells activation status.

This combined bioassay, kit or device comprises necessary elements for obtaining an immunocapture of the enzyme that is present in the biological sample, by an enzyme recognizing (polyclonal or monoclonal) primary antibody or a portion thereof, which are both specific of the said enzyme and fixed upon a solid support surface.

Advantageously, this combined bioassay (kit or device) further comprises a specific substrate to be transformed by the enzyme into a visible reaction product, (preferably a fluorescent reaction product) for a detection of the enzymatically active fraction of the immunocaptured enzyme, and a (possibly labeled) (polyclonal or monoclonal) secondary antibody (or a portion thereof) for a detection of the total amount (level or value) of immunocaptured enzyme by the primary antibody.

Preferably this combined bioassay (kit or device) further comprises a calibrated (for enzymatic activity and for total amount) reference enzyme sample to use for establishing reference curves. Furthermore, the kit or device may further comprise data, possibly presented in a database or upon graphics (calibration or standard curve), related to normal (enzymatically active or total) enzyme amount (levels or values) obtained from a significant number (more than 10, preferably more than 50, more preferably more than 200 or 1000 individuals) of "healthy" mammals preferably horses or human (i.e. mammals that present normal MPO levels and do not suffer from the following described diseases or symptoms).

These normal enzyme levels are advantageously present in a standard MPO curve or are collected in a database. Therefore, the kit or device may further comprise these databases stored on a computer (with adequate software) to compare the measured enzyme (levels or values) with normal enzyme amount (levels or values) by method well known by the person skilled in the art and to relate these measured enzyme (levels or values) to an activity status of the enzyme or an activation status of the cells releasing the enzyme, this status being possibly indicative of the presence, absence or condition of a disease or immunological status of mammal subjects, preferably horses or human.

In the method according to the invention, the enzyme is preferably selected from the group consisting of myeloperoxidase (MPO), or an enzyme being marker of an inflammation pathology or a cardiac pathology (preferably arteriosclerosis pathology) or cancer.

Preferably, this enzyme is selected from the group consisting of proteases (trypsin, elastase,...), NADPH oxidase, glutathione peroxidase, NO synthase, catalase or collagenases.

In the method according to the invention, the solid support is preferably a multiwells plate, preferably a streptavidin coated multiwells plate which allow the binding of the primary antibody (or a portion thereof) to its surface. Preferably, this coating is sufficient to avoid removal of this coated primary antibody after (first and/or second) washing step(s) used to eliminate from the biological sample any molecule which is not bounded to the primary antibody or a portion thereof. Furthermore, this coating is also sufficient to avoid any removal of this primary antibody (or a portion thereof) by a washing step used to eliminate the enzyme substrate after the SIEFED detection step and before the addition of the (labeled) second antibody further used for the ELISA detection step.

Furthermore, the bioassay, kit or device disclosed may further comprise any other means or media used to perform the combined detection according to the invention, such as nitrite(s) which is added to the reaction medium to amplify enzymatic reaction, preferably enzymatic reaction of peroxidase, more preferably to enhance 10-acetyl-3,7-dihydroxyphenoxazine induced fluorescence signal.

It was surprisingly found that the detection sensitivity of the SIEFED method steps could be significantly increased by the addition of nitrites which significantly enhanced fluorescence. The preferred amount of nitrite (NO₂⁻) to add to the reaction mixture is comprised between about 0.05 to about 0.7 mg/ml and preferably is about 0.2 mg/ml. Nitrite is preferably added under the form of a salt such as a Na-salt or any other alkali or earth alkali salt (such as Li, K, Rb, Cs, Be, Mg, Ca, Sr salts) except toxic salts (such as presumably Ba, Ra or Fr salts). Amplification of the detection signal makes it possible to accurately measure and detect the enzymatic activity of an enzyme, for instance that of MPO originating from neutrophils, in the most complex (biological) media, tissues or samples.

In a particular and preferred embodiment of the invention, the sensitivity of the enzymatic detection was increased at least 2-fold, preferably at least 5-fold, most preferably at least 10-fold or 20-fold by using nitrite as peroxidase activity enhancer leading to an enhanced fluorescence response.

This technique of fluorescence enhancement is equally well applicable to a detection of other peroxidase activities, and is applicable not only to the described SIEFED methods, kits and devices, but to any (medical or industrial) detection method or kit that may require the use of a peroxidase enzyme.

The bioassay, kit or device disclosed may also further comprise other well known means and media, such as chromogene(s), buffer(s), label(s), and washing solution(s). Furthermore, the kit or device according to the invention may also comprise detection and/or recording means and media of the signal(s) obtained from the used label(s).

The biological sample or medium is preferably a (possibly purified) biological fluid obtained from a mammal (preferably a horse or a human) or a culture medium or obtained from bacteria plants or fungi, including yeast cells that may release the enzyme. This biological fluid could be a cellular biological fluid or an acellular biological fluid. This biological fluid could be venous and capillary blood, serum or plasma, seminal fluid, bronchoalveolar fluid, pleural fluid, sputum, nasal fluid, ascites fluids, synovial fluid, gastric bowel and faecal derivate samples or cerebrospinal fluid.

The biological sample or medium could also be an extract obtained from various tissues of a mammal or other complex biological samples or media which may also comprise other molecules, such as proteins (albumin, lipoprotein) and reducing agents that may interfere with adequate enzyme measurement as observed for classical tests known in the art.

The methods according to the invention also apply to some specific diagnostic assays already proposed for horses or humans, such as the detection of diseases of inflammatory origin, which may affect said mammal, especially a horse or a human.

In a preferred aspect of the disclosure, the kit (or device) is kits-of-parts (possibly comprising different parts of the elements for performing the method steps).

The potential applications of a combined SIEFED-ELISA method according to the invention are:
- the rapid evaluation of the intensity of cells (neutrophil cells) activation and systemic or local inflammatory reaction, in acute or chronic inflammation pathologies (such as atherosclerosis, sepsis, septic shock, pulmonary inflammation pathologies, intestinal pathologies, laminitis).
- the efficient and rapid follow-up of the activation of cells (neutrophil cells) during therapy (study of the effects of drugs administrated to the mammal subject (preferably a horse or a human), taking samples of the same diseased individual or mammal (preferably a horse or a human) at different time intervals whereby the neutrophil activation status can be followed in time.
- the early diagnostic or forecasting of some pathologies.
- the rapid evaluation of the ability of neutrophil cells)to destroy micro-organisms (evaluation on isolated neutrophils), a test to be applied in immunosuppression pathologies.
- the rapid evaluation of the natural defense capacity or ability of an individual or group of individuals to fight against infections.
- the rapid measurement of enzyme capture by other cells (in relation with their ability to fight against micro-organisms and/or to destroy them).
- The rapid screening and the selection of compounds, possibly interacting with the enzyme and possibly affecting the activity of this enzyme.

Therefore, the kit or device disclosed is preferably a high throughput screening kit or device which comprises elements for measuring, screening, selecting active compounds. Such 'active compounds' are elements selected from the group consisting of chemically or biologically synthesized molecules (including antibodies), purified new natural molecules, microorganism plants or animal extracts or a mixture thereof. These compounds are preferably enzyme inhibitors (reversible or irreversible inhibitors). Preferably said kit or device used for screening enzyme inhibitors is based upon a method which comprises the steps of
- incubation the active enzyme with the potential inhibitor(s),
- capturing active enzyme (preferably via enzyme specific antibody or a hypervariable portion thereof) bound to a solid support, preferably the solid support of the SIEFED-ELISA kit or device according to the invention, and washing the unbound compounds,
- possibly measuring enzyme activity, preferably by the SIEFED step (part) of the SIEFED-ELISA method above described,
- washing to eliminate the reagents after the SIEFED step of the SIEFED-ELISA method,
- possibly measuring the total amount of enzyme, preferably by the ELISA step (part) of the SIEFED-ELISA method above described,
- possibly comparing enzyme activity and total enzyme before or after addition of the potential inhibitor(s).

When examining the ability of cells other than neutrophils to fight against micro-organisms and/or to destroy them, the bioassays that have been described above are then applied to samples containing other cells type. By comparing enzyme levels obtained for said cells with neutrophil enzyme levels of the healthy individuals, an estimate can be made of the capacity or ability of said cells to fix or to internalize the enzyme and consequently to fight infections by micro-organisms.

The above detection techniques can further find advantageous use in the study of the efficiency of certain medicaments such as immunomodulators. Enzyme levels of (neutrophils) cells that have been in contact with for instance said immunomodulators are then compared with enzyme amount (levels or values) of non-treated (neutrophils) cells, said enzyme (levels or values) being an indication for the cells (neutrophil cells) activation status and/or their ability to fight and/or destroy micro-organisms.

The detection methods according to the invention are in particular useful in the prediction, the diagnosis, possibly in a very early stage, and/or the follow-up of one of the following pathologies or diseases: inflammatory diseases, digestive pathologies, strangulated intestinal pathologies, sepsis, septic shock, chronic and acute pulmonary pathologies (with invasion of the alveoli by neutrophils), ischemia-reperfusion pathologies, articular pathologies (with presence of neutrophils in the joints), colics, allergies, infections, cardiovascular diseases and cancer.

The detection methods according to the invention are further particularly useful for the *in vitro* evaluation of the inhibitory capacity on enzyme activity of drugs (either natural products obtained from plant extracts or from animal origin, or newly synthesized molecules), allowing to distinguish between a neutralizing effect of said drugs on the products of enzyme activity (stoechiometric anti-oxidant activity) or a direct inhibitory activity on the enzyme function itself (anti-catalytic activity).

In a more general way, by comparing the enzymatic activity levels and the total enzyme levels measured via the combined SIEFED-ELISA steps, the efficiency of enzyme purification techniques can be assayed.

The present invention will now be described in detail in the following description of preferred embodiments of the present invention in reference to the enclosed figures.

The examples given below are offered by way of illustration, not by way of limitation.

### Short description of the figures

The figure 1 represents two standard or calibration curves used in the method according to the invention.

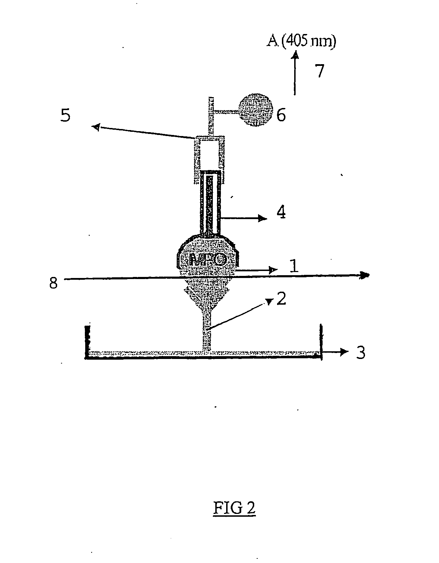

The figure 2 represents the combined method of the invention.

### Examples

### Example 1 : Combined SIEFED-ELISA for human MPO

### a) Preparation and purification of human MPO antibodies

The polyclonal antibodies specific against human MPO were raised in rabbits and guinea pigs against pure human MPO, purified by affinity chromatography and tested for immunoreactivity following the same methods as described for the preparation and purification of polyclonal anti-equine MPO.

### b) Combined SIEFED-ELISA technique to measure human MPO

The methods used are similar to those described for the combined SIEFED-ELISA of equine MPO, but specific polyclonal anti-human MPO antibodies (rabbit IgG for the primary antibody and guinea-pig IgG for the second antibody) are used.

### c) Calibration with human MPO

Calibration curves for the combined SIEFED-ELISA were obtained with standard human MPO:
- as used in CORD laboratory: a stock solution (100µg/ml) of human MPO (Calbiochem) in a sodium acetate buffer 0,2M, pH 5,5 is maintained into aliquots fractions at -20°C. This stock solution is diluted with a dilution buffer to reach a concentration of 50 ng/ml of MPO (concentration of Calibrator 7 (Cal 7)). Starting from Cal 7 the sequential dilutions are obtained until reaching the lowest MPO concentration for the curve: 0,78 ng/ml (Cal 1). The Calibrator 0 (Cal 0) = dilution buffer.
- as used in the Zentech ELISA kit for human MPO:
   MPO Calibrators of ELISA kit are prepared according to the recommended mode provided by Zentech. The concentrations of MPO are (in ng/ml : 0 (Cal 0); 0,6 (Cal 1); 2,1 (Cal 2); 11,7 (Cal 3); 22,4 (Cal 4); 78,5 (Cal 5). To these calibrators are added two internal plasmatic references having the following estimated concentration of total MPO:
      Control 1 : 1,20 (0,80 ... 2, 00) ng/ml
      Control 2: 8,50 (6,30 ... 10, 70) ng/ml

### d) Sample dilution

The dilution of the sample is obtained with the dilution buffer provided in the Zentech kit. The biological samples are diluted 20x for the combined SIEFED-ELISA dosage.

### e) Enzyme standard curves for the combined SIEFED-ELISA

An enzyme (human MPO) standard curve was obtained for the SIEFED part of the combined SIEFED-ELISA after revelation of the enzymatic activity, by plotting the fluorescence values at 590 nm as a function of the standard enzyme concentrations. The "SIEFED part" standard curve allows to quantify the enzymatically active fraction of MPO in a biological sample.

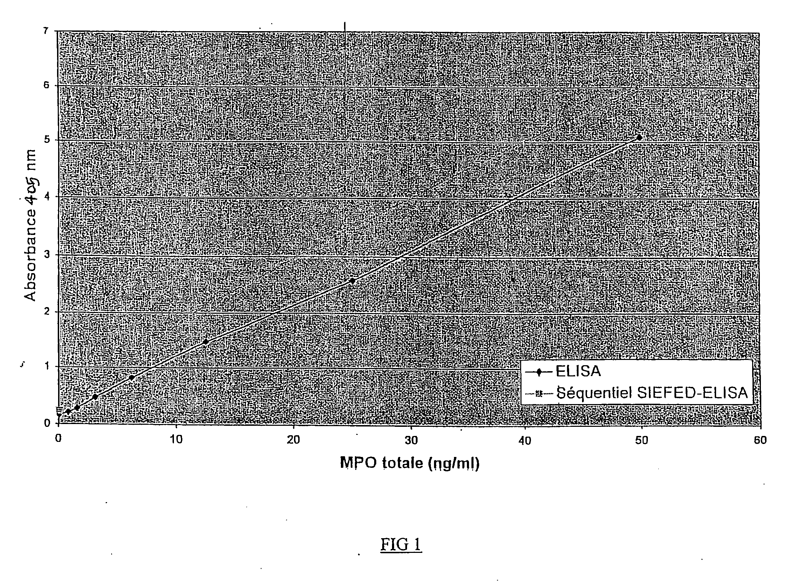

The standard curve of the ELISA part of the combined SIEFED-ELISA was obtained by plotting the absorbance values at 405 nm as a function of the standard enzyme concentrations (see figure 1). The "ELISA part" standard curve allows to quantify the total concentration of MPO in a biological sample. The ratio total MPO/active MPO in the biological sample is obtained by the ratio of the two values read on the two standard curves.

### f) Comparative analysis of active fraction and total enzyme (human MPO) concentration measured in tested biological samples: SIEFED/ELISA concentrations ratio.

The table 1 presents concentration of an enzyme (human MPO) (total and active) measured in the controls of the Zentech kit, in plasmas (P) and bronchoalveolar liquid (Bal) of human.

By using a suitable human MPO calibrator from CORD for the SIEFED-ELISA detection, the Inventors have observed that active enzyme (MPO) concentrations are always lower (at least 2 to 3 x) than the total enzyme (MPO) concentration in a tested biological samples (see table 1).

**Table 1:**

| Calibration CORD MPO (Calbiochem) | ELISA Total MPO (ng/ml) | SIEFED Active MPO (ng/ml) | Total MPO/active MPO |
|---|---|---|---|
| Control MPO 1 | 1,36 | 0,37 | 3,6 |
| Control MPO 2 | 11,65 | 0,63 | 18,5 |
| P5 20 x | 58,30 ± 2,6 | 25,57 | 2,3 |
| P6 20 x | 35,25 ± 2,2 | 12,34 | 2,9 |
| Bal De 20 x | 13,76 ± 3,5 | 6,78 | 2,0 |
| Bal Ha 20 x | ≥ 50 | 10,41.55 | ≥ 5 |

These results show that the combined SIEFED-ELISA bioassay (kit or device) for human MPO is efficient and allows to measure sequentially the enzymatically active fraction and the total amount of MPO on the same biological sample with the same primary antibody(2) (or the same hypervariable portion thereof) fixed upon a solid support surface(3). Indeed, the Inventors have observed that the biological sample can be used with the same dilution for the SIEFED and ELISA (20 x in the present bioassay). This means that for the same diluted sample, active fraction only and total enzyme (MPO) level are in concentrations fitting with the sensitivity limit of the bioassay and the reference curves, because they can be measured with the same dilution factor by using the same immunological material (primary antibody) and the same calibrators. These advantageous characteristics therefore clearly simplify the methods steps according to the invention compared to the separated ELISA and SIEFED bioassays.

These dilution factors could be modified according to the type of enzyme and according to the type of biological sample.

### Example 2 : Combined SIEFED-ELISA for equine MPO

### a) Preparation and purification of equine MPO antibodies

Production of polyclonal antibodies are described in the W02005/075986 incorporated herein by reference.

Raised antibodies efficiently recognize the enzyme (MPO)

### b) Combined SIEFED-ELISA technique to measure equine MPO released by actived neutrophils.

SIEFED is an immunodetection technique consisting of two steps:
- the capture of an enzyme (such as MPO) (1) from biological samples by a specific primary antibody (2) coated onto a solid support (3), followed by
- the enzymatic detection of the enzyme (such as MPO) (1) immobilized (fixed) on the solid support-coated primary antibody.

Contrary to the ELISA steps (part) of the combined test, the SIEFED (part) measures active enzyme (MPO) only.

The primary antibody(2), that captures the enzyme (MPO 1), is a rabbit IgG (3µg/ml). Standard enzyme (MPO) or unknown sample is incubated 2 h at 37°C. After washing, the (peroxidase) activity of the enzyme (MPO) is detected by adding 100 µl of a 10 µM H₂O₂ solution and 40 µM of Amplex® Red (8) (10-acetyl-3,7-dihydroxyphenoxazine; from Molecular Probes) in phosphate buffer (50 mM, pH 7.5). The fluorescence is read during 30 min (at 37°C) at 590 nm with a Fluoroskan Ascent apparatus (Labsystems). The volumes of the primary antibody(2) and the sample put in the wells, are 200 µl. Controls (blank) and dilutions of the samples are established with dilution buffer.

Enhancement of fluorescence was obtained when adding a defined concentration of nitrites ions (about 10 mM) to the Amplex® Red solution (8). The fluorescence response obtained in this SIEFED steps (part) of the combined SIEFED-ELISA bioassay is directly proportional to the enzymatically active fraction of the enzyme (equine MPO) present in the sample.

After the SIEFED detection, a washing (0.9 % NaCl solution containing 0.1 % tween 20) is performed before starting the ELISA part of the combined method; the immobilized antibody(2)-antigen(1) complex is incubated (2 h, 37°C) with an excess (5 µg/ml) of guinea pig IgG, the secondary antibody (4). After washing, a third antibody (5) produced against guinea pig IgG is added. This third IgG (5) (goat IgG) is labelled with alkaline phosphatase (6) and recognizes the "sandwich" complex "primary antibody (2)-MPO(1)-secondary antibody"(4). After washing, phosphatase activity is detected by incubation (30 min, 37°C, in the darkness) with a paranitrophenyl phosphate solution (phosphatase substrate, Sigma). The reaction is stopped with NaOH and the absorbance (405 nm) is measured with a Multiscan Ascent apparatus (Labsystem). All the volumes added into the wells comprise 100 µl, except for washing (300 µl) and for the substrate solution (200 µl). Controls (blank) and dilutions of the standard enzyme (MPO) and samples were established with dilution buffer [PBS (20 mM phosphate, 137 mM NaCl and 2.7 mM KCl pH 7.4) to which 5g/L bovine serum albumin and 0.1 % tween 20 were added].

The absorbance response obtained for this ELISA part of the combined method is directly proportional to the quantity of sandwich complex formed, in other words to the total concentration of enzyme (MPO) in the sample.

### c) Enzyme standard curves for the combined SIEFED-ELISA bioassay for equine MPO

An enzyme (equine MPO) standard curve was obtained for the SIEFED steps (part) of the combined SIEFED-ELISA by plotting the fluorescence values at 590 nm as a function of the standard enzyme concentrations and for the ELISA steps (part) of the combined SIEFED-ELISA by plotting the absorbance values at 405 nm as a function of standard enzyme (MPO) concentrations. The standard curves are classical ones, reaching a plateau for the highest enzyme (MPO) concentrations. Linear curves are obtained when enzyme (MPO) concentrations are expressed in the logarithmic form. A table (see W02005/075986) lists the fluorescence and absorbance values with standard deviations (SD) and intra-assay variation coefficients (CV in %) for the SIEFED and ELISA reference curve of the combined SIEFED-ELISA.

Enzyme (MPO) standard curves allow quantification of enzymatically active fraction of the enzyme (SIEFED standard curve) and the total amount of enzyme (MPO)(ELISA standard curve). The rate of total to active enzyme is obtained by the rate of the values read on the respective standard curves. Monitoring of disease progression benefits from such quantification. By comparing mean active and total enzyme (MPO) levels of healthy with diseased individuals, cut-off values can be established that allow distinction between healthy and diseased mammals. Preferably such cut-off values are established for the different biological samples assayed for cells enzyme (MPO) levels.

The addition of nitrite ions (about 10 mM) under the form of a salt (sodium salt) to the reaction medium could enhance until tenfold the sensibility of the SIEFED steps (part) of the SIEFED-ELISA bioassay.

### d) Developed combined SIEFED-ELISA bioassay for equine MPO allows detection of enzyme in complex samples such as plasma and supernatant of activated neutrophils

Active and total enzyme (MPO) levels were detected in biological samples consisting of plasma drawn from blood anticoagulated on EDTA, as it was previously demonstrated that EDTA was the best anticoagulant avoiding in vitro degranulation of neutrophils in the blood sample and artefactual values of active and total MPO concentrations.

Intra -and inter- assay coefficients of variation (witness of the precision of the technique) were established for plasma taken from healthy horses and horses with inflammation pathologies.

The highest concentrations of active and total enzyme (MPO) were observed in plasma from horses with intestinal strangulated pathologies.
An important liberation of total enzyme (MPO) with variable fraction of enzymatically active enzyme was observed in the supernatant of excited neutrophils in comparison to non-excited ones.

The above demonstrates that the combined SIEFED-ELISA bioassay of the present invention is sensitive, accurate and clearly able to make a distinction between healthy and pathological animals. They further demonstrate that the measurement of plasma total and active enzyme (MPO) can be taken as the witness of cells (neutrophils) activation and are positively correlated to certain pathologies.

The combined test was also applied with success to peritoneal fluids and seminal liquids.

### e) Sensitivity and precision of the developed combined SIEFED-ELISA bioassay

The sensitivity of the combined assay is about 1 ng/ml, with good intra- and inter-assay precisions for standard curves (inferior to 8 %) and biological samples (generally inferior to 10 %).

### Example 3 : Combined SIEFED-ELISA for the study of the effects of the natural polyphenols, curcumin and tetrahydrocurcumin (THC) on activated equine neutrophils

Neutrophils isolated from citrated blood of healthy horses were counted, suspended in phosphate buffer saline (PBS) at pH 7.4 and incubated 10 min at 37°C with curcumin or THC at the final concentration of 10⁻⁴, 10⁻⁵ or 10⁻⁶ M. After incubation and centrifugation (1000 x g, 10 min), the neutrophils were resuspended in PBS and activated 30 min with 8 x10⁻⁷ M phorbol myristate acetate (PMA) followed by centrifugation and active and total enzyme (MPO) measurement in the supernatant by the combined SIEFED-ELISA specific bioassay. The effects of polyphenols on total and active fraction of enzyme (MPO) released by stimulated neutrophils were studied by pre-incubation of neutrophils with the drugs at various concentrations, before the neutrophil stimulation.

Curcumin and THC both had dose dependent inhibitory effects on the total enzyme, and on the active fraction of the enzyme (MPO) released by activated neutrophils. The inhibition percentages were 44 % and 60 % for curcumin (10-5 M) and 18 %, and 22 % for THC (10-5 M) on total enzyme (MPO) release and on MPO activity respectively. These inhibitory effects of curcumin arise therapeutic perspectives in equine pathologies with excessive inflammatory reactions.

The enzymatic activity of enzyme (MPO) produces HOCl (hypochlorous acid) or NaOCl (sodium hypochlorite) and other oxidant species potentially toxic if the enzyme acts directly in contact with tissues or into the cells, thus in places other than in the phagolysosome. Enzyme (MPO) can be present in biological fluids in an inactive form (inhibition by oxidation or by specific inhibitors). It is interesting to distinguish the active enzyme (MPO) from its inactive form in biological samples.

## Claims

1. An *in vitro* method for the sequential measurement of firstly the enzymatically active fraction and secondly the total amount of an enzyme(1) in the same complex biological sample, said method being a combination of a Specific Immunological Extraction Followed by Enzymatic Detection or SIEFED method and an Enzyme-linked Immuno Sorbent Assay or ELISA method, **characterized in that** this "combined SIEFED-ELISA" method being performed upon the same solid support with the same primary antibody(2) or hypervariable portion of primary antibodyboth being specific of the said enzyme and fixed upon the solid support surface (3),

2. The method according to the claim 1 which comprises the successive step of :
1) immunocapturing an enzyme (1) present in a biological sample obtained from a mammal, said sample containing the enzyme (1) or cells which may release the said enzyme by specific polyclonal or monoclonal primary antibody (2) or a hypervariable portion thereof, both being specific of the said enzyme(1) and being fixed upon a solid support surface (3),
2) removing by a first washing step any compound that interferes with the measurement of the enzymatic activity of the enzyme immunocapturated upon the solid support (3) surface by the primary antibody (2) or its portion,
3) obtaining from a SIEFED detection and quantification by detecting and measuring the immunocaptured enzyme (1) activity, a measured enzyme activity value,
4) possibly comparing the said measured enzyme activity value with normal enzyme activities values obtained from a significant number of healthy mammals
5) and optionally quantifying the enzyme activity value by using a standard enzyme curve,
6) removing by a second washing step any substrate and compound used for or resulting from the SIEFED steps detection and present in the solution upon the immunocapturated enzyme (1),
7) obtaining from an ELISA. detection a measured total enzyme amount by detecting and measuring total active and inactive amount of the enzyme (1) present in a biological sample, by an addition of a polyclonal or monoclonal secondary antibody (4) or a portion thereof, for a detection of the enzyme (1) immunocaptured by the said primary antibody (2) or its portion,
8) possibly comparing the said measured total enzyme amount with normal total enzyme amount obtained from a significant number of healthy mammals,
9) optionally quantifying the total enzyme amount by using standard enzyme curve and possibly relating the total enzyme amount measured to an activation status of the cell releasing the enzyme in relation with the presence, absence or condition of a disease or immunological status,
10) possibly calculating the ratio of total enzyme to active enzyme in a biological sample and comparing this ratio to a normal ratio obtained from a significant number of healthy mammals, or relating this ratio to an activation status of the enzyme linked to a disease condition.

3. The method according to the claim 2 wherein the step of obtaining a SIEFED detection and quantification by detecting and measuring the enzyme activity is obtained by adding a specific substrate(8) to be transformed by the enzyme (1) into a visible reaction product.

4. The method of claim 3, wherein the visible reaction product is a fluorescent reaction product.

5. The method according to claims 1 to 4, wherein the biological sample is obtained from a mammal.

6. The method of claim 5 wherein the mammal is a horse or a human.

7. The method according to any of the preceding claims, wherein the enzyme is selected from the group consisting of myeloperoxidase, proteases NADPH oxidase, glutathione peroxidase, NO synthase, catalase or collagenases.

8. The method of claim 7, wherein the protease is trypsin or elastase

9. The method according to any of the preceding claims, wherein the solid support (3) is a multiwell plate.

10. The method according to any of the preceding claims, wherein the secondary antibody (4) is labeled,

11. The method according to the claim 10, wherein the secondary antibody (4) is labelled by a coupling to an enzyme.

## Patentansprüche

1. In vitro-Verfahren für das sequentielle Messen von erstens der enzymatisch aktiven Fraktion und zweitens des Gesamtgehalts eines Enzyms (1) in derselben komplexen, biologischen Probe, wobei das Verfahren eine Kombination eines Specific Immunologial Extraction Followed by Enzymatic Detection- oder SIEFED-Verfahrens und eines Enzyme-limked Immuno Sorbent Assay- oder ELISA-Verfahrens darstellt, **dadurch gekennzeichnet, dass** dieses "Combined SIEFED-ELISA"-Verfahren auf demselben festen Träger mit demselben primären Antikörper(2)- oder hypervariablen Anteil von primärem Antikörper, wobei beide spezifisch für das Enzym sind und auf der festen Trägeroberfläche (3) immobilisiert sind, ausgeführt wird.

2. Verfahren nach Anspruch 1, welches den nachfolgenden Schritt umfasst:
1) immunologisches Abfangen eines Enzyms (1), welches in einer biologischen Probe vorhanden ist, die aus einem Säugetier erhalten wird, wobei die Probe das Enzym (1) oder Zellen enthält, welche das Enzym durch spezifische polyklonale oder monoklonale Antikörper (2) oder einen hypervariablen Anteil davon freisetzen können, wobei beide für das Enzym (1) spezifisch sind und auf einer festen Trägeroberfläche (3) immobilisiert sind,
2) Entfernen durch einen ersten Waschschritt jeglicher Verbindung, welche die Messung der enzymatischen Aktivität des Enzyms stört, das immunologisch auf der Oberfläche des festen Trägers (3) durch den primären Antikörper (2) oder seinen Anteil abgefangen wurde,
3) Erzielen, ausgehend von der SIEFED-Erkennung und Mengenerfassung durch Erkennen und Messen der Aktivität der immunologisch abgefangenen Enzyme (1), eines gemessenen Enzymaktivitätswertes,
4) möglichst Vergleichen des gemessenen Enzymaktivitätswertes mit normalen Enzymaktivitätswerten, welche von einer signifikanten Anzahl gesunder Säuger erhalten wurde,
5) und wahlweise Messen des Enzymaktivitätswertes durch Einsatz einer Standardenzymkurve,
6) Entfernen durch einen zweiten Waschschritt jeglichen Substrats und jeglicher Verbindung, welche für die Erkennung durch die SIEFED-Schritte verwendet wurden oder sich aus diesen ergaben und in der Lösung auf dem immunologisch abgefangenen Enzym (1) vorhanden sind,
7) Erzielen, ausgehend von einer ELISA-Erkennung, eines gemessenen Gesamtenzymgehalts durch Erfassen und Messen des aktiven und inaktiven Gesamtgehalts des Enzyms (1), welcher in einer biologischen Probe vorhanden ist, durch eine Zugabe eines polyklonalen oder monoklonalen Antikörpers (4) oder eines Anteils davon für eine Erkennung des Enzyms (1), das immunologisch durch den primären Antikörper (2) oder seinen Anteil abgefangen wurde,
8) möglichst Vergleichen des gemessenen Gesamtenzymgehalts mit dem normalen Enzymaktivitätsspiegel, welcher von einer signifikanten Anzahl gesunder Säuger erhalten wurde,
9) wahlweise Messen des Gesamtenzymgehalts durch Verwenden der Standardenzymkurve und möglichst Inbeziehungsetzen des gemessenen Gesamtenzymgehalts mit einem Aktivitätszustand der Zellen, welche das Enzym in Bezug auf das Vorhandensein, auf das Fehlen oder auf einen Krankheitszustand oder immunologischen Zustand freisetzen,
10) möglichst Berechnen des Verhältnisses von Gesamtenzym zu aktivem Enzym in einer biologischen Probe und Vergleichen dieses Verhältnisses zu einem normalen Verhältnis, welches von einer signifikanten Anzahl gesunder Säuger erhalten wurde, oder Inbeziehungsetzen dieses Verhältnisses zu einem Aktivitätszustand des Enzyms, welcher mit einem Krankheitszustand verbunden ist.

3. Verfahren nach Anspruch 2, wobei der Schritt des Erzielens einer SIEFED-Erfassung und Messung durch Erfassen und Messen der Enzymaktivität durch Hinzufügen eines spezifischen Substrats (8) erzielt wird, welches durch das Enzym (1) in ein sichtbares Reaktionsprodukt umgewandelt wird.

4. Verfahren nach Anspruch 3, wobei das sichtbare Reaktionsprodukt ein fluoreszierendes Reaktionsprodukt ist.

5. Verfahren nach den Ansprüchen 1 bis 4, wobei die biologische Probe von einem Säuger erhalten wird.

6. Verfahren nach Anspruch 5, wobei der Säuger ein Pferd oder ein Mensch ist.

7. Verfahren nach jedem der vorangehenden Ansprüche, wobei das Enzym aus der Gruppe ausgewählt ist, welche Myeloperoxidase, Peptidasen NADPH-Oxidase, Glutathion-Peroxidase, NO-Synthase, Katalase oder Collagenase umfasst.

8. Verfahren nach Anspruch 7, wobei die Peptidase Trypsin oder Elastase ist.

9. Verfahren nach jedem der vorangehenden Ansprüche, wobei der feste Träger (3) eine Multiwell-Platte ist.

10. Verfahren nach jedem der vorangehenden Ansprüche, wobei der sekundäre Antikörper (4) gekennzeichnet ist.

11. Verfahren nach Anspruch 10, wobei der sekundäre Antikörper (4) durch Koppeln an ein Enzym gekennzeichnet ist.

## Revendications

1. Méthode *in vitro* pour la mesure séquentielle premièrement de la fraction enzymatiquement active et deuxièmement de la quantité totale d'une enzyme (1) dans le même échantillon biologique complexe, ladite méthode étant une combinaison d'une extraction immunologique spécifique suivie d'une détection enzymatique ou méthode SIEFED et d'un dosage d'immunoadsorption par enzyme liée ou méthode ELISA, **caractérisée en ce que** cette méthode "SIEFED-ELISA combinée" est mise en oeuvre sur le même support solide avec le même anticorps primaire (2) ou la même partie hypervariable d'anticorps primaire, l'un et l'autre étant spécifiques de ladite enzyme et immobilisés sur la surface du support solide (3).

2. Méthode selon la revendication 1, qui comprend les étapes successives consistant à :
1) immunocapturer une enzyme (1) présente dans un échantillon biologique prélevé chez un mammifère, ledit échantillon contenant l'enzyme (1) ou des cellules capables de sécréter ladite enzyme, au moyen d'un anticorps primaire polyclonal ou monoclonal spécifique (2) ou d'une partie hypervariable de cet anticorps, l'un et l'autre étant spécifiques de ladite enzyme (1) et immobilisés sur la surface d'un support solide (3),
2) éliminer par une première étape de lavage un éventuel composé qui perturbe la mesure de l'activité de l'enzyme immunocapturée sur la surface du support solide (3) au moyen de l'anticorps ou de la partie d'anticorps primaire (2),
3) obtenir par une détection et quantification SIEFED, en détectant et/ou mesurant l'activité de l'enzyme immunocapturée (1), une valeur d'activité enzymatique mesurée,
4) le cas échéant, comparer ladite valeur d'activité enzymatique mesurée avec des valeurs d'activité enzymatique normale obtenues à partir d'un nombre significatif de mammifères sains,
5) et, facultativement, quantifier la valeur d'activité enzymatique en utilisant une courbe d'enzyme étalon,
6) éliminer par une deuxième étape de lavage le substrat et le composé éventuels utilisés pour ou résultant des étapes de détection SIEFED et présents dans la solution sur l'enzyme immunocapturée (1),
7) obtenir par une détection ELISA une quantité totale d'enzyme mesurée, en détectant et mesurant la quantité totale active et inactive de l'enzyme (1) présente dans un échantillon biologique, par l'ajout d'un anticorps secondaire polyclonal ou monoclonal (4) ou d'une partie de cet anticorps, pour une détection de l'enzyme (1) immunocapturée par ledit anticorps ou ladite partie d'anticorps primaire (2),
8) le cas échéant, comparer ladite quantité totale d'enzyme mesurée à la quantité d'enzyme totale normale obtenue à partir d'un nombre significatif de mammifères sains,
9) facultativement, quantifier la quantité d'enzyme totale en utilisant une courbe d'enzyme étalon et le cas échéant corréler la quantité d'enzyme totale mesurée à un état d'activation de la cellule qui sécrète l'enzyme en rapport avec la présence, l'absence ou la condition d'une maladie ou d'un état immunologique,
10) le cas échéant, calculer le ratio de l'enzyme totale sur l'enzyme active dans un échantillon biologique et comparer ce ratio à un ratio normal obtenu à partir d'un nombre significatif de mammifères sains, ou corréler ce ratio à un état d'activation de l'enzyme associée à une condition pathologique.

3. Méthode selon la revendication 2, dans laquelle l'étape d'obtention d'une détection et quantification SIEFED en détectant et mesurant l'activité enzymatique se fait en ajoutant un substrat spécifique (8) destiné à être transformé par l'enzyme (1) en un produit réactionnel visible.

4. Méthode selon la revendication 3, dans laquelle le produit réactionnel visible est un produit réactionnel fluorescent.

5. Méthode selon l'une quelconque des revendications 1 à 4, dans laquelle l'échantillon biologique est prélevé chez un mammifère.

6. Méthode selon la revendication 5, dans laquelle le mammifère est un cheval ou un être humain.

7. Méthode selon l'une quelconque des revendications précédentes, dans laquelle l'enzyme est choisie dans le groupe constitué par la myéloperoxydase, les protéases, la NADPH oxydase, la glutathion peroxydase, la NO synthétase, la catalase ou les collagénases.

8. Méthode selon la revendication 7, dans laquelle la protéase est la trypsine ou l'élastase.

9. Méthode selon l'une quelconque des revendications précédentes, dans laquelle le support solide (3) est une plaque multipuits.

10. Méthode selon la revendication 7, dans laquelle l'anticorps secondaire (4) est marqué.

11. Méthode selon la revendication 10, dans laquelle l'anticorps secondaire (4) est marqué par conjugaison à une enzyme.
